# EUROPEAN PATENT SPECIFICATION

(11) **EP 3 159 847 B1**
(45) Date of publication and mention of the grant of the patent: **29.04.2020**
(21) Application number: 15821955.0
(22) Date of filing: 09.07.2015
(51) Int. Cl.: G16H 10/60, G16H 80/00, G06Q 50/22, G06F 21/62

(54) **INFORMATION SHARING SYSTEM, PATIENT TERMINAL, AND INFORMATION MANAGEMENT DEVICE**
SYSTEM ZUR GEMEINSAMEN NUTZUNG VON INFORMATIONEN, PATIENTENENDGERÄT UND INFORMATIONSVERWALTUNGSVORRICHTUNG
SYSTÈME DE PARTAGE D'INFORMATIONS, TERMINAL DE PATIENT, ET DISPOSITIF DE GESTION D'INFORMATIONS

(30) Priority: 17.07.2014 JP 2014146828
(43) Date of publication of application: 26.04.2017
(73) Proprietor: Allm Inc., Tokyo 150-0002 (JP)
(72) Inventor: SAKANO Teppei, Tokyo 150-0002 (JP); TAKAO Hiroyuki, Tokyo 150-0001 (JP)
(74) Representative: Stöckeler, Ferdinand
(86) International application number: PCT/JP2015/069740
(87) International publication number: WO 2016/009934

(56) References cited:
- WO-A1-2014/100036
- WO-A2-2007/050877
- WO-A2-2014/113817
- JP-A- 2002 073 822
- JP-A- 2003 076 789
- JP-A- 2012 248 027
- US-A1- 2009 070 146
- US-A1- 2009 204 434
- US-A1- 2012 095 779
- US-A1- 2012 102 559

## Description

### TECHNICAL FIELD

The present invention relates to an information sharing system, a patient terminal, and an information management apparatus.

### BACKGROUND ART

The following personal information disclosure server is known. In the personal information disclosure server, an information terminal of an access source is permitted access to personal information if the location of the information terminal approaches the whereabouts of a person to whom the personal information belongs (refer to Patent Document 1).

Patent Document 2 discloses means for improving delivery of healthcare services while protecting patient privacy, comprising providing Portable Medical Assistant Device (PMAD), a Home Base Device (HBD), and a Central Program and Management System (CPMS).

### CITATION LIST

### PATENT LITERATURE

Patent Document 1: JP-A-2012-248027
Patent Document 2: WO 2014/100036 A1

### SUMMARY OF THE INVENTION

### PROBLEMS TO BE SOLVED BY THE INVENTION

A known personal information disclosure server assumes a case where doctors and caregivers view personal information of patients and the like outside a hospital. Therefore, if an information terminal carried by a doctor or caregiver approaches the whereabouts of a person to whom personal information belongs, the information terminal is permitted access to the personal information; accordingly, security is increased. However, when a patient visits a facility such as a hospital, the patient himself/herself may desire to disclose his/her personal information and share it with a doctor or a worker involved in care work at a place where he/she visits. In this case, the personal information needs to be disclosed to the worker of the patient's own will. However, a technology for increasing security in the known personal information disclosure server in such a case has not been studied at all.

### SOLUTIONS TO THE PROBLEMS

An information sharing system according to a first aspect of the present invention is an information sharing system including: a worker terminal carried by a worker involved in health care or care work; a mobile patient terminal carried by a patient who permits the disclosure of his/her personal information to the worker; and an information management apparatus connected to the worker terminal and the patient terminal via a communications line. The mobile patient terminal includes: a patient terminal current location identifying unit for identifying the current location of the patient terminal; a disclosure request accepting unit for accepting a request of the patient to disclose the personal information to the worker; a worker terminal current location identifying unit for, upon the disclosure request accepting unit accepting the disclosure request, receiving information indicating the current location of the worker terminal from the information management apparatus, and identifying the current location of the worker terminal; a permission determination unit for determining whether or not to permit the worker as a disclosure destination of the personal information based on the current location of the patient terminal identified by the patient terminal current location identifying unit and the current location of the worker terminal identified by the worker terminal current location identifying unit; and a disclosure permission information transmission unit for, upon the permission determination unit having determined to permit the worker as the disclosure destination of the personal information, transmitting, to the information management apparatus, disclosure permission information indicating that the personal information of the patient has been permitted to be disclosed to the worker. The information management apparatus includes: a worker terminal current location information receiving unit for receiving information indicating the current location of the worker terminal from the worker terminal at predetermined time intervals; a worker terminal current location information transmission unit for transmitting, to the patient terminal, the information indicating the current location of the worker terminal received by the worker terminal current location information receiving unit, wherein a worker terminal current location information transmission unit transmits the information indicating the current location of the worker terminal received by the worker terminal current location information receiving unit, when the current location of the worker terminal is the closest location to the patient terminal, a disclosure permission information receiving unit for receiving the disclosure permission information from the patient terminal; and a personal information transmission unit for, upon the disclosure permission information receiving unit having received the disclosure permission information, transmitting the personal information of the patient recorded in a recording medium to the worker terminal of the worker to whom the personal information of the patient has been permitted to be disclosed. The worker terminal includes: a worker terminal current location identifying unit for identifying the current location of the worker terminal at predetermined time intervals, and transmitting information indicating the identified current location to the information management apparatus; a personal information receiving unit for receiving the personal information of the patient from the information management apparatus; and a personal information display unit for displaying the personal information of the patient received by the personal information receiving unit.

According to a second aspect of the present invention, in the information sharing system of the first aspect, when the current location of the patient terminal and the current location of the worker terminal are within a predetermined distance, the permission determination unit determines to permit the worker as the disclosure destination of the personal information.

According to a third aspect of the present invention, in the information sharing system of the first or second aspect, when the current location of the patient terminal and the current location of the worker terminal are located within a site of a preset medical institution, the permission determination unit determines to permit the worker as the disclosure destination of the personal information.

According to a fourth aspect of the present invention, in the information sharing system of the third aspect, when a carrier of the worker terminal is a person who belongs to the medical institution, the permission determination unit determines to permit the worker as the disclosure destination of the personal information.

According to a fifth aspect of the present invention, in the information sharing system of the fourth aspect, when the personal information is permitted to be disclosed to the carrier of the worker terminal in the medical institution, the permission determination unit determines to permit the worker as the disclosure destination of the personal information.

According to a sixth aspect of the present invention, in the information sharing system of the fourth or fifth aspect, the personal information includes referral information for a doctor referring the patient to another doctor, established specialty department identification-purpose information for identifying specialty departments established in the medical institution to which the carrier of the worker terminal belongs is recorded in the information management apparatus, and the permission determination unit refers to the established specialty department identification-purpose information, and upon having determined that the medical institution has a department corresponding to a specialty specified by the referral information, determines to permit the worker as the disclosure destination of the personal information.

According to a seventh aspect of the present invention, in the information sharing system of the sixth aspect, worker specialty department identification-purpose information for identifying a specialty department of the carrier of the worker terminal is recorded in the information management apparatus, and the permission determination unit refers to the worker specialty department identification-purpose information, and upon having determined that a specialty department corresponding to the specialty specified by the referral information agrees with the specialty department of the carrier of the worker terminal, determines to permit the worker as the disclosure destination of the personal information.

According to an eighth aspect of the present invention, in the information sharing system of the sixth or seventh aspect, when the medical institution includes a facility in accordance with the purpose of the referral specified by the referral information, the permission determination unit determines to permit the worker as the disclosure destination of the personal information.

According to a ninth aspect of the present invention, in the information sharing system of the eighth aspect, admission facility possession/non-possession information indicating the possession/non-possession of an admission facility by the medical institution to which the carrier of the worker terminal belongs is recorded in the information management apparatus, and when the purpose of the referral specified by the referral information is transfer, the permission determination unit refers to the admission facility possession/non-possession information, and upon having determined that the medical institution has an admission facility, determines to permit the worker as the disclosure destination of the personal information.

According to a tenth aspect of the present invention, in the information sharing system of the eighth or ninth aspect, surgical facility possession/non-possession information indicating the possession/non-possession of a surgical facility by the medical institution to which the carrier of the worker terminal belongs is recorded in the information management apparatus, and when the purpose of the referral specified by the referral information is surgery, the permission determination unit refers to the surgical facility possession/non-possession information, and upon having determined that the medical institution has a surgical facility, determines to permit the worker as the disclosure destination of the personal information.

According to an eleventh aspect of the present invention, in the information sharing system of any of the eighth to tenth aspects, photographic facility possession/non-possession information indicating the possession/non-possession of a photographic facility by the medical institution to which the carrier of the worker terminal belongs is recorded in the information management apparatus, and when the purpose of the referral specified by the referral information is photography, the permission determination unit refers to the photographic facility possession/non-possession information, and upon having determining that the medical institution has a photographic facility, determines to permit the worker as the disclosure destination of the personal information.

According to a twelfth aspect of the present invention, in the information sharing system of any of the first to eleventh aspects, the patient terminal further includes a consent confirmation unit for, when the permission determination unit has determined to permit the worker as the disclosure destination of the personal information, confirming the patient's consent to the disclosure of the personal information to the worker, and when the consent confirmation unit has confirmed the patient's consent to the disclosure of the personal information, the disclosure permission information transmission unit transmits the disclosure permission information to the information management apparatus.

According to a thirteenth aspect of the present invention, the mobile patient terminal is used in the above information sharing system.

According to a fourteenth aspect of the present invention, the information management apparatus is used in the above information sharing system.

### ADVANTAGEOUS EFFECTS OF THE INVENTION

According to the present invention, a patient who wants to permit disclosure of his/her personal information to a worker who is involved in health care or care work can disclose the personal information to the worker of his/her own will and also can increase security in the disclosure of the personal information by determining whether or not to permit the worker as a disclosure destination of the personal information based on the current location of a patient terminal and the current location of a worker terminal.

### BRIEF DESCRIPTION OF THE DRAWINGS

Fig. 1 is a block diagram illustrating the configuration of one embodiment of an information sharing system 10.
Fig. 2 is a block diagram illustrating the configuration of one embodiment of a patient terminal 100.
Fig. 3 is a block diagram illustrating the configuration of one embodiment of a worker terminal 200.
Fig. 4 is a block diagram illustrating the configuration of one embodiment of an information management apparatus 300.
Fig. 5 is a flowchart diagram illustrating the flow of processes to be executed by the patient terminal 100.
Fig. 6 is a flowchart diagram illustrating the flow of processes to be executed by the worker terminal 200.
Fig. 7 is a flowchart diagram illustrating the flow of processes to be executed by the information management apparatus 300.

### DESCRIPTION OF EMBODIMENTS

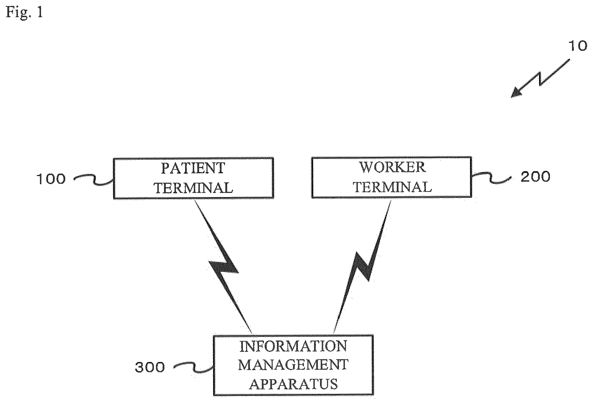

Fig. 1 is a block diagram illustrating the configuration of one embodiment of an information sharing system according to the embodiment. An information sharing system 10 includes a patient terminal 100, a worker terminal 200, and an information management apparatus 300. The patient terminal 100 is a terminal carried by a patient who permits disclosure of his/her personal information to a worker who is involved in health care or care work. For example, an information terminal such as a smartphone or tablet terminal is used as the patient terminal 100. The worker terminal 200 is a terminal carried by a worker who is involved in health care or care work. For example, an information terminal such as a smartphone or tablet terminal is used as the worker terminal 200. The information management apparatus 300 is an apparatus that is connected to the patient terminal 100 and the worker terminal 200 via a communications line, and communicates with the patient terminal 100 and the worker terminal 200 to execute various processes. For example, an information processing apparatus such as a server apparatus or personal computer is used as the information management apparatus 300.

In the embodiment, a health care worker such as a doctor, a nurse, or a clerk of a medical institution to which the doctor and the nurse belong, or a care worker such as a caregiver or a clerk of a care institution to which the caregiver belongs is assumed as the worker. Moreover, a patient requiring care who receives a diagnosis, treatment, and nursing care from the health care workers, and also receives care from the care workers is assumed as the patient.

In the embodiment, the patient terminal 100, the worker terminal 200, and the information management apparatus 300 are connected via a communications line such as the Internet or a mobile phone network. Fig. 1 illustrates an example where the information sharing system 10 includes one patient terminal 100, one worker terminal 200, and one information management apparatus 300. However, the configuration of the information sharing system 10 is not limited to this. For example, processing may be distributed to a plurality of information management apparatuses 300, and the numbers of the patient terminals 100 and the worker terminals 200 are not particularly limited.

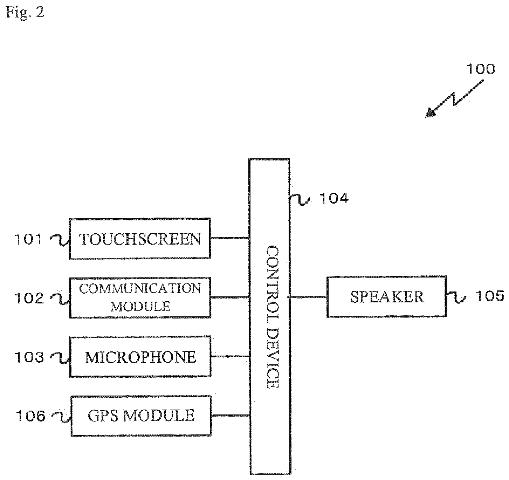

Fig. 2 is a block diagram illustrating the configuration of one embodiment of a case where a smartphone is used as the patient terminal 100 according to the embodiment. In the embodiment, an application aimed at the patient for communicating with the information management apparatus 300, and providing various functions described below is installed in the smartphone; accordingly, the smartphone operates as the patient terminal 100 according to the embodiment. The following is assumed to be performed in advance: a program of the application aimed at the patient is provided by being disclosed on a server connected via the Internet to allow a user to download and obtain the program and install it in the patient terminal 100. Alternatively, the program of the application aimed at the patient may be provided by being recorded in a recording medium such as a CD-ROM or DVD-ROM.

The patient terminal 100 includes a touchscreen 101, a communication module 102, a microphone 103, a control device 104, a speaker 105, and a GPS module 106.

The touchscreen 101 is an electronic component where a display device such as a liquid crystal panel and a position input device such as a touchpad are combined, and is an input device that can operate equipment by pressing a display on the screen. For example, the patient being a user of the patient terminal 100 uses the finger or a touch pen to touch or slide a displayed item such as a button or menu displayed on the liquid crystal panel, and accordingly can operate the patient terminal 100. The touchscreen 101 detects an operation such as a touch or a slide by the patient to output the detection signal to the control device 104.

A module for connecting to the Internet via a LAN or a mobile phone communications network by wired or wireless is used for the communication module 102. The patient terminal 100 communicates with the information management apparatus 300 via the communication module 102.

The microphone 103 is a sound collecting device for inputting the voice uttered by the patient. Analog data input from the microphone 103 is converted into a digital signal by the control device 104.

The control device 104 includes a CPU, memory, and other peripheral circuits, and controls the entire patient terminal 100. The memory included in the control device 104 includes volatile memory such as SDRAM and nonvolatile memory such as flash memory. The volatile memory is used as work memory for expanding a program when the CPU executes the program, and as buffer memory for recording data temporarily. Moreover, firmware for operating the patient terminal 100 and program data of software for operating various applications are recorded in the nonvolatile memory. In the embodiment, the program of the above-mentioned application aimed at the patient is recorded in the nonvolatile memory.

The speaker 105 is an output device for outputting voice. The control device 104 outputs, via the speaker 105, voice data of when there is an incoming call from another user and voice data recorded in the nonvolatile memory.

The GPS module 106 is a module for communicating with GPS satellites and receiving GPS signals.

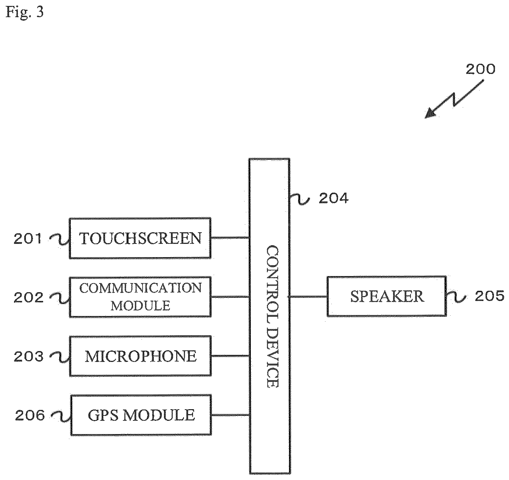

Fig. 3 is a block diagram illustrating the configuration of one embodiment of when a smartphone is used as the worker terminal 200 according to the embodiment. In the embodiment, an application aimed at the worker for communicating with the information management apparatus 300 and providing various functions described below is installed in the smartphone; accordingly, the smartphone operates as the worker terminal 200 according to the embodiment. The following is assumed to be performed in advance: a program of the application aimed at the worker is provided by being disclosed on a server connected via the Internet to allow a user to download and obtain the program and install it in the worker terminal 200. Alternatively, the program of the application aimed at the worker may be provided by being recorded in a recording medium such as a CD-ROM or DVD-ROM.

The worker terminal 200 includes a touchscreen 201, a communication module 202, a microphone 203, a control device 204, a speaker 205, and a GPS module 206.

The touchscreen 201 is an electronic component where a display device such as a liquid crystal panel and a position input device such as a touchpad are combined, and is an input device that can operate equipment by pressing a display on the screen. For example, the worker being a user of the worker terminal 200 uses the finger or a touch pen to touch or slide a displayed item such as a button or menu displayed on the liquid crystal panel, and accordingly can operate the worker terminal 200. The touchscreen 201 detects an operation such as a touch or a slide by the worker to output the detection signal to the control device 204.

A module for connecting to the Internet via a LAN or a mobile phone communications network by wired or wireless is used for the communication module 202. The worker terminal 200 communicates with the information management apparatus 300 via the communication module 202.

The microphone 203 is a sound collecting device for inputting the voice uttered by the worker. Analog data input from the microphone 203 is converted into a digital signal by the control device 204.

The control device 204 includes a CPU, memory, and other peripheral circuits, and controls the entire worker terminal 200. The memory included in the control device 204 includes volatile memory such as SDRAM and nonvolatile memory such as flash memory. The volatile memory is used as work memory for expanding a program when the CPU executes the program, and as buffer memory for recording data temporarily. Moreover, firmware for operating the worker terminal 200 and program data of software for operating various applications are recorded in the nonvolatile memory. In the embodiment, the program of the above-mentioned application aimed at the worker is recorded in the nonvolatile memory.

The speaker 205 is an output device for outputting voice. The control device 204 outputs, via the speaker 205, voice data of when there is an incoming call from another user and voice data recorded in the nonvolatile memory.

The GPS module 206 is a module for communicating with GPS satellites and receiving GPS signals.

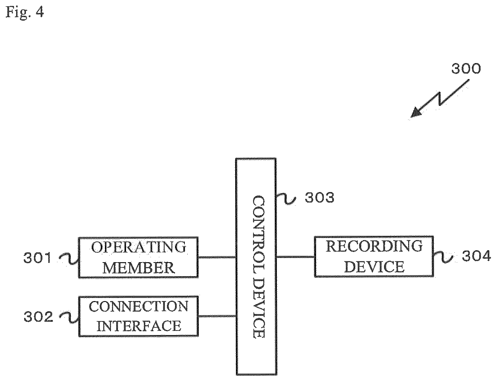

Fig. 4 is a block diagram illustrating the configuration of one embodiment of when a server apparatus is used as the information management apparatus 300 according to the embodiment. The information management apparatus 300 includes an operating member 301, a connection interface 302, a control device 303, and a recording device 304.

The operating member 301 includes various devices, such as a keyboard and a mouse, that are operated by an operator of the information management apparatus 300.

The connection interface 302 is an interface for connecting the information management apparatus 300 to a communications line such as the Internet. For example, a wired LAN module for connecting to the Internet by wire or a wireless LAN module for connecting to the Internet wirelessly is used as the connection interface 302. The information management apparatus 300 communicates with the patient terminal 100 and the worker terminal 200 via the connection interface 302.

The control device 303 includes a CPU, memory, and other peripheral circuits, and controls the entire information management apparatus 300. The memory included in the control device 303 is volatile memory such as SDRAM. The memory is used as work memory for expanding a program when the CPU executes the program, and as buffer memory for recording data temporarily. For example, data read in via the connection interface 302 is recorded temporarily in the buffer memory.

The recording device 304 is a recording medium for recording, for example, various kinds of data stored in the information management apparatus 300 and data of a program to be executed by the control device 303. For example, an HDD (Hard Disk Drive) or SSD (Solid State Drive) is used as the recording device 304. The data of the program recorded in the recording device 304 is provided by being recorded in a recording medium such as a CD-ROM or DVD-ROM. Alternatively, the data of the program recorded in the recording device 304 is provided via a network. A user installs, in the recording device 304, the data of the program provided in this manner; accordingly, it becomes possible for the control device 303 to execute the program.

In the information sharing system 10 according to the embodiment, the patient who carries the patient terminal 100 can disclose his/her own personal information to the worker who carries the worker terminal 200 through the information sharing system 10.

In the embodiment, upon use of the information sharing system 10, patient information related to the patient, belonged institution information related to an institution to which the worker belongs, and worker information related to the worker are recorded in advance in the recording device 304 of the information management apparatus 300. In the following description, a case is assumed where a health care worker and a care worker are workers, an institution to which the health care worker belongs is a hospital, and an institution to which the care worker belongs is a care provider. The patient information related to the patient, hospital information related to the hospital, care provider information related to the care provider, and health care worker and care worker information related to the health care worker and the care worker are assumed to be recorded in advance in the recording device 304 of the information management apparatus 300.

The patient information includes the patient's basic information, login information of the application aimed at the patient, the patient's life information, the patient's treatment information, the patient's handoff information, and the patient's care and nursing information. In the embodiment, the application aimed at the patient has a function of accepting the input and editing of the patient's basic information and life information. When the patient inputs or edits these pieces of information on the patient terminal 100, the control device 104 transmits the input or edited information to the information management apparatus 300. The patient's basic information and life information are then recorded in the recording device 304. Moreover, the application aimed at the worker has a function of accepting the input and editing of the patient's treatment information, handoff information, and care and nursing information. When the worker inputs or edits these pieces of information on the worker terminal 200, the control device 204 transmits the input or edited information to the information management apparatus 300. The patient's treatment information, handoff information, and care and nursing information are then recorded in the recording device 304.

The patient's basic information includes various kinds of information required to allow the patient to receive health care and care. For example, information such as the patient's name, gender, birthdate, address, telephone number, email address, occupation, blood type, height, weight, medications taken, medical history and disease names, allergy names, family medical institution name, family doctor name, emergency contact information, and insurance information is recorded in advance as the patient's basic information. For example, a family's telephone number is recorded as the emergency contact information. Information such as the insured number, insurer number, and insurer name of an insurance card is recorded as the insurance information.

The patient's life information includes information related to the everyday life of the patient. For example, the patient's family information, names of family members living together, information indicating an assistance requiring state, information indicating outpatient treatment, information indicating home treatment, and information indicating a care plan is recorded in advance as the patient's life information. For example, information such as the family structure, the names of the family members, contact information of the family is recorded as the patient's family information. Details of assistance that is required by the patient in everyday life, such as meals, toileting, and bathing, are recorded as the information indicating an assistance requiring state. Information such as the name of a hospital that the patient visits on a regular basis, the name of the attending doctor, and details of treatment is recorded as the information indicating outpatient treatment. Information such as the name of a hospital in charge of home treatment and the name of a visiting doctor is recorded as the information indicating home treatment. Information indicating the contents of services that the patient receives on a plan basis or limit basis, such as home-visit care, home-visit bathing service, home-visit nursing care, daycare rehabilitation service, the use of welfare equipment, living support, an assistance facility, and a medical facility is recorded as the information indicating a care plan.

The login information of the application aimed at the patient includes information that is necessary for the patient to log in from the patient terminal 100 to the information sharing system 10. For example, information such as a mobile phone number of the patient, and a MAC address and password of the patient terminal 100 is recorded as the login information of the application aimed at the patient.

The patient's treatment information includes information related to the details of treatment for the patient and a result of the treatment. For example, the patient's basic information, the name of a hospital that provides treatment, an attending specialty department, the name of a doctor, the name of the current disease or injury, the current primary symptoms, the latest examination findings, follow-up of treatment, prescription contents, a family history, a medical history, and vitals information of the patient are registered as the patient's treatment information. The name, gender, birthdate, and address of the patient are recorded as the patient's basic information. Information such as the body temperature, blood pressure, pulse, respirations, and blood glucose level is recorded on an observation date and time basis as the patient's vitals information.

The patient's handoff information includes information related to the patient to be handed off between health care workers or care workers responsible for the patient. For example, the patient's handoff information includes referral information and medical image data. The referral information includes information in accordance with the content of the description of a referral that is created when a doctor refers the patient to another doctor. For example, information indicating the purpose of the referral, information related to a referral source, the patient's basic information, the information related to the details of treatment for the patient, information related to the specialty required in treatment of the patient, information related to the family history and medical history of the patient, information indicating whether or not a referral destination is asked for an answer, and hand over information from the referral source to the referral destination are registered as the referral information. Information indicating the purpose of the referral - admission, transfer, surgery, intensive examination, treatment, or others - is recorded as the information indicating the purpose of the referral. Information such as a medical institution name, attending specialty department, address, telephone number, fax number, and name of an attending doctor of the referral source medical institution is recorded as the information related to the referral source. The name, gender, birthdate, address, and the like of the patient are recorded as the patient's basic information. Information such as the name of the current disease or injury, the current primary symptoms, the latest examination findings, follow-up of treatment, and prescription contents is recorded as the information related to the details of treatment for the patient. Information for identifying a specialty such as neurology, obstetrics and gynecology, or cardiac surgery is recorded as the information related to the specialty required in treatment of the patient. Moreover, various kinds of image data such as a CT image, an MRI image, an ultrasound image, and an X-ray image, which were taken during the process of the examination and treatment of the patient are recorded as the medical image data.

The care and nursing information of the patient includes information related to the care and nursing of the patient. For example, information on the care required, information indicating the content of a care plan, information indicating the level of cognition, information related to a care manager in charge, information indicating the care plan, information related to the schedule of the care and nursing, and the patient's vitals information are recorded as the care and nursing information of the patient. Information such as the certified level of long-term care required and the certified period is recorded as the information on the care required. The name of the care manager in charge, the name of an institution to which the care manager in charge belongs, contact information of the care manager in charge, and the like are recorded as the information related to a care manager in charge. The information indicating the contents of services that the patient receives on a plan basis or limit basis, such as home-visit care, home-visit bathing service, home-visit nursing care, daycare rehabilitation service, the use of welfare equipment, living support, an assistance facility, and a medical facility, is recorded as the information indicating a care plan as in the above description. Information indicating the frequency, time, unit, number of times, a person in charge, and the like of each care plan service is recorded as the information related to the schedule of the care and nursing. Information such as the body temperature, blood pressure, pulse, respirations, and blood glucose level is recorded on an observation date and time basis as the patient's vitals information as in the above description.

The hospital information is input by a hospital that uses the information sharing system 10 to be transmitted to the information management apparatus 300, and accordingly recorded in advance in the recording device 304. The hospital information is assumed to be input in a terminal installed in each hospital and transmitted to the information management apparatus 300. In the embodiment, the hospital information input in a terminal installed in each hospital is managed by a management system that is introduced independently by the hospital. A description is given here of an example where a management system of each hospital is linked to the information sharing system 10 to transmit, to the information management apparatus 300, the hospital information registered in a server forming the management system of the hospital, and accordingly is registered in the recording device 304.

The hospital information includes the name of a hospital, specialty departments, a provider ID uniquely assigned to the hospital, the possession/non-possession of an admission facility, the possession/non-possession of a surgical facility, the possession/non-possession of a photographic facility, the address of the hospital, the telephone number, the fax number, and information on the latitude and longitude of the site, and authentication information for authenticating the management system of the hospital. The authentication information for authenticating the management system of the hospital is information that is used to allow the information management apparatus 300 to authenticate the management system of the hospital upon linking the management system of the hospital to the information sharing system 10. The authentication information for authenticating the management system of the hospital includes, for example, connection destination information such as an IP address and MAC address of a server to which the information management apparatus 300 permits a connection, and information related to encryption such as an encrypted format and an encryption key.

The care provider information is input by a care provider that uses the information sharing system 10 to be transmitted to the information management apparatus 300, and accordingly recorded in advance in the recording device 304. The care provider information is assumed to be input in a terminal installed in each care provider and transmitted to the information management apparatus 300. In the embodiment, the care provider information input in a terminal installed in each care provider is managed by a management system that is introduced independently by the care provider. A description is given here of an example where a management system of each care provider is linked to the information sharing system 10 to transmit, to the information management apparatus 300, the care provider information registered in a server forming the management system of the care provider, and accordingly is registered in the recording device 304.

The care provider information includes the name of a care provider, the contents of services provided by the care provider, a provider ID uniquely assigned to the care provider, and authentication information for authenticating the management system of the care provider. Information indicating the contents of services provided by the care provider, such as home-visit care, home-visit bathing service, home-visit nursing care, and daycare rehabilitation service, is registered as the contents of services provided by the care provider. Moreover, the authentication information for authenticating the management system of the care provider is information that is used to allow the information management apparatus 300 to authenticate the management system of the care provider upon linking the management system of the care provider to the information sharing system 10. The authentication information for authenticating the management system of the care provider includes, for example, connection destination information such as an IP address and MAC address of a server to which the information management apparatus 300 permits a connection, and information related to encryption such as an encrypted format and an encryption key.

The health care worker and care worker information includes information on the name of a health care worker/care worker, the login information from the application aimed at the worker, the specialty of the health care worker/care worker, the name of the hospital or care provider to which the health care worker/care worker belongs, the qualification held, and the permissions assigned. Information on the qualification held by each worker, such as a doctor, a nurse, and a caregiver, is recorded as the qualification held. Moreover, information indicating patient information view and edit permissions assigned to the health care worker by the medical institution to which he/she belongs, and information indicating patient information view and edit permissions assigned to the care worker by a care institution to which he/she belongs are recorded as the permissions assigned. In the embodiment, the application aimed at the worker has a function of accepting the input and edit of the health care worker and care worker information. When the worker inputs or edits these pieces of information on the worker terminal 200, the control device 204 transmits the input or edited information to the information management apparatus 300; accordingly, the health care worker and care worker information is recorded in the recording device 304.

In the information sharing system 10 according to the embodiment, the patient who receives health care and nursing care starts the application aimed at the patient on the patient terminal 100 to log in to the information sharing system 10 with the above-mentioned login information of the application aimed at the patient; accordingly, it becomes possible to use the information sharing system 10 via the application aimed at the patient. For example, if the MAC address and password of the patient terminal 100 is used as the login information, when the patient instructs the start of the application aimed at the patient, the control device 104 displays, on the touchscreen 101, a login screen that prompts the input of the password, and accepts the patient's input of the password on the login screen. When the patient has input the password, the control device 104 transmits, to the information management apparatus 300, a pair of the MAC address of the patient terminal 100 and the password input by the patient as login requirement information.

When having received the login requirement information from the patient terminal 100 in the information management apparatus 300, the control device 303 determines whether or not there is information where the pair of the MAC address and the password included in the login information of the application aimed at the patient agrees with the pair of the MAC address and the password included in the login requirement information received from the patient terminal 100, in the patient information recorded in the recording device 304. If there is the information that agrees as a result, then a login permission signal is transmitted to the patient terminal 100. On the other hand, if there is not the information that agrees, then a login reject signal is transmitted to the patient terminal 100. Moreover, the control device 303 identifies patient information where the pair of the MAC address and the password included in the login information of the application aimed at the patient agrees with the pair of the MAC address and the password included in the login requirement information, as the patient information of the patient who has been permitted a login.

When having received the login permission signal from the information management apparatus 300 in the patient terminal 100, the control device 104 permits the patient to use the application aimed at the patient. When having received the login reject signal from the information management apparatus 300, the control device 104 rejects the patient's use of the application aimed at the patient. Similarly, the worker starts the application aimed at the worker on the worker terminal 200 to log in to the information sharing system 10 with the above-mentioned login information of the application aimed at the worker; accordingly, it becomes possible to use the information sharing system 10 via the application aimed at the worker.

When desiring to disclose his/her own patient information to a specific worker, the patient who has been permitted to use the application aimed at the patient operates the patient terminal 100, and accordingly can instruct the disclosure of his/her own patient information to the specific worker to share the information.

For example, the following case is conceivable as the case where the patient desires to disclose his/her own patient information to a specific worker: it is assumed that in a situation where the patient visits a certain hospital A on a regular basis, the patient is referred to another hospital B by a doctor of the hospital A. In this case, the patient's treatment information and handoff information, which were input by the doctor of the hospital A, have been recorded in the recording device 304. The patient is to disclose the patient information including the treatment information and the handoff information to an attending doctor of the hospital B when visiting the hospital B. Consequently, the patient's treatment information and handoff information are shared between the doctor of the hospital A and the doctor of the hospital B.

In order to achieve this, the patient operates the patient terminal 100 in front of a worker to whom the patient desires to disclose his/her own patient information to perform a predetermined operation for an information disclosure instruction. The operation for an information disclosure instruction may be, for example, an operation of touching a button for instructing the disclosure of the information, the button being displayed on the touchscreen 101, or may be an operation of, for example, holding and swinging the patient terminal 100 with the application aimed at the patient started.

When having detected the patient's operation for an information disclosure instruction, the control device 104 identifies the current location, for example, the latitude and longitude, of the patient terminal 100 based on GPS signals received by the GPS module 106, and transmits information indicating the identified current location to the information management apparatus 300.

When having received the current location information from the patient terminal 100 in the information management apparatus 300, the control device 303 identifies the worker terminal 200 being the closest to the patient terminal 100 based on the received current location information. In order to achieve this, each worker terminal 200 transmits the information indicating the current location of the worker terminal 200 to the information management apparatus 300 at predetermined time intervals, for example, at intervals of 10 minutes. The control device 303 records the latest information indicating the current location received from each worker terminal 200 in the buffer memory or recording device 304. The control device 303 then compares the information indicating the current location received from the patient terminal 100 with the information indicating the current locations of the worker terminals 200 recorded in the buffer memory or recording device 304 at a timing when receiving the current location information from the patient terminal 100. The control device 303 identifies the worker terminal 200 being the closest to the patient terminal 100. The control device 303 then transmits, to the patient terminal 100, the information indicating the current location of the identified worker terminal 200 being the closest to the patient terminal 100.

When having received the information indicating the current location of the worker terminal 200 from the information management apparatus 300 in the patient terminal 100, the control device 104 executes a permission determination process for determining whether or not to permit a worker who carries the worker terminal 200 as the disclosure destination of the personal information, that is, the patient information, of the patient based on the current location of the patient terminal 100 and the current location of the worker terminal 200

A description is given here of the permission determination process by the control device 104. The control device 104 executes the following permission determination process steps (A) to (G), and judges whether or not to permit the disclosure of the patient information to the worker who carries the worker terminal 200 whose information indicating the current location has been received from the information management apparatus 300, based on the execution results. As described below, the permission determination process steps (B) to (G) assume a case where the worker is a health care worker. Accordingly, if the worker is a care worker, whether or not to permit the disclosure of the patient information is judged based only on the result of the execution of the permission determination process step (A).

In the permission determination process step (A), if the current location of the patient terminal 100 and the current location of the worker terminal 200 are within a predetermined distance, for example, within 10 m, the control device 104 determines to permit the worker who carries the worker terminal 200 as the disclosure destination of the patient information. On the other hand, if the above condition is not satisfied, the control device 104 determines to not permit the worker who carries the worker terminal 200 as the disclosure destination of the patient information. Specifically, the control device 104 calculates a distance between the patient terminal 100 and the worker terminal 200 by a known method, for example, using a trigonometric function to calculate a distance between two points based on the latitude and longitude of the patient terminal 100 and the latitude and longitude of the worker terminal 200. When the distance is within the predetermined distance that has been preset as a threshold, the control device 104 determines to permit the worker who carries the worker terminal 200 as the disclosure destination of the patient information.

In the permission determination process step (B), if the current location of the patient terminal 100 and the current location of the worker terminal 200 are located within a site of a preset medical institution, the control device 104 determines to permit the worker who carries the worker terminal 200 as the disclosure destination of the patient information. On the other hand, if the above condition is not satisfied, the control device 104 determines to not permit the worker who carries the worker terminal 200 as the disclosure destination of the patient information. Specifically, the control device 104 refers to the health care worker and care worker information of the worker to whom the disclosure of the patient information was determined to be permitted in the permission determination process step (A) to identify the name of a hospital to which the worker belongs, and also refers to the above-mentioned hospital information to identify the latitude and longitude of a site of the hospital. When the current location of the worker terminal 200 of the worker to whom the disclosure of the patient information was determined to be permitted in the permission determination process step (A) and the current location of the patient terminal 100 are located within the area of the site of the hospital identified based on the latitude and longitude of the site, then the control device 104 determines to permit the worker who carries the worker terminal 200 as the disclosure destination of the patient information. It is simply required to use information that enables substantially accurate identification of the area of the site, such as information on the latitudes and longitudes of a plurality of points forming the boundaries of the site or information on the latitude and longitude of each vertex point of the site, as the information on the latitude and longitude of the site included in the hospital information.

In the permission determination process step (C), if the carrier of the worker terminal 200 belongs to the medical institution, the control device 104 determines to permit the worker who carries the worker terminal 200 as the disclosure destination of the patient information. On the other hand, if the above condition is not satisfied, the control device 104 determines to not permit the worker who carries the worker terminal 200 as the disclosure destination of the patient information. Specifically, the control device 104 refers to the health care worker and care worker information of the worker to whom the disclosure of the patient information was determined to be permitted in the permission determination process step (B), and if the name of the hospital to which the worker belongs agrees with the name of the medical institution corresponding to the area of the site of the medical institution where the current location of the worker terminal 200 was located in the permission determination process step (B), determines to permit the worker who carries the worker terminal 200 as the disclosure destination of the patient information.

In the permission determination process step (D), if the patient information is permitted to be disclosed to the carrier of the worker terminal 200 in the medical institution, the control device 104 permits the worker who carries the worker terminal 200 as the disclosure destination of the patient information. On the other hand, if the above condition is not satisfied, the control device 104 determines to not permit the worker who carries the worker terminal 200 as the disclosure destination of the patient information. Specifically, the control device 104 refers to the health care worker and care worker information of the worker to whom the disclosure of the patient information was determined to be permitted in the permission determination process step (B), checks the permission assigned to the worker, and if permission to view the patient information is assigned to the worker, determines to permit the worker who carries the worker terminal 200 as the disclosure destination of the patient information.

In the permission determination process step (E), if the medical institution to which the worker belongs has a department corresponding to the specialty specified by the information related to the specialty required in treatment of the patient, the information being included in the referral information, the control device 104 permits the worker who carries the worker terminal 200 as the disclosure destination of the patient information. On the other hand, if the above condition is not satisfied, the control device 104 determines to not permit the worker who carries the worker terminal 200 as the disclosure destination of the patient information. Specifically, the control device 104 refers to the referral information included in the patient's handoff information and identifies the specialty required in treatment of the patient. Furthermore, the control device 104 refers to the health care worker and care worker information of the worker to whom the disclosure of the patient information was determined to be permitted in the permission determination process step (B) to identify the name of the hospital to which the worker belongs, and refers to the hospital information to identify the specialty departments established in the hospital having the above hospital name. If the specialty departments established in the hospital include a specialty department corresponding to the specialty required in treatment of the patient, the specialty department being identified by referring to the referral information, then the control device 104 determines to permit the worker who carries the worker terminal 200 as the disclosure destination of the patient information.

In the permission determination process step (F), if the specialty specified by the information related to the specialty required in treatment of the patient, the information being included in the referral information, agrees with a specialty of the carrier of the worker terminal 200, the control device 104 determines to permit the worker who carries the worker terminal 200 as the disclosure destination of the patient information. On the other hand, if the above condition is not satisfied, the control device 104 determines to not permit the worker who carries the worker terminal 200 as the disclosure destination of the patient information. Specifically, the control device 104 refers to the referral information included in the patient's handoff information to identify the specialty required in treatment of the patient. Furthermore, the control device 104 refers to the health care worker and care worker information of the worker to whom the disclosure of the patient information was determined to be permitted in the permission determination process step (B) to identify a specialty department of the worker. If the specialty department of the worker agrees with a specialty department corresponding to the specialty required in treatment of the patient, the specialty being identified by referring to the referral information, the control device 104 determines to permit the worker who carries the worker terminal 200 as the disclosure destination of the patient information.

In the permission determination process step (G), if the medical institution includes a facility in accordance with the purpose of the referral, the facility being specified by the referral information, the control device 104 permits the worker who carries the worker terminal 200 as the disclosure destination of the patient information. On the other hand, if the above condition is not satisfied, the control device 104 determines to not permit the worker who carries the worker terminal 200 as the disclosure destination of the patient information. Specifically, if the purpose of the referral that is identified based on the information indicating the purpose of the referral, the information being included in the referral information, is transfer, the control device 104 refers to the hospital information of the hospital to which the worker belongs, the worker having been determined to be permitted to be disclosed the patient information in the permission determination process step (B), and if the hospital has an admission facility, determines to permit the worker who carries the worker terminal 200 as the disclosure destination of the patient information. Moreover, if the purpose of the referral that is identified based on the information indicating the purpose of the referral, the information being included in the referral information, is surgery, the control device 104 refers to the hospital information of the hospital to which the worker belongs, the worker having been determined to be permitted to be disclosed the patient information in the permission determination process step (B), and if the hospital has a surgical facility, determines to permit the worker who carries the worker terminal 200 as the disclosure destination of the patient information. Moreover, if the purpose of the referral that is identified based on the information indicating the purpose of the referral, the information being included in the referral information, is photography, the control device 104 refers to the hospital information of the hospital to which the worker belongs, the worker having been determined to be permitted to be disclosed the patient information in the permission determination process step (B), and if the hospital has a photographic facility, determines to permit the worker who carries the worker terminal 200 as the disclosure destination of the patient information.

If having determined to permit the disclosure of the patient information to the worker targeted for the permission determination process as the execution results of the permission determination process steps (A) to (G), the control device 104 transmits, to the information management apparatus 300, disclosure permission information indicating that the patient information has been permitted to be disclosed to the worker. The disclosure permission information includes information for identifying the patient whose patient information has been permitted to be disclosed, such as the name of the patient, and information for identifying the worker who has been permitted as the disclosure destination, such as the name of the health care worker/care worker.

When having received the above disclosure permission information from the patient terminal 100 in the information management apparatus 300, the control device 303 reads the patient information of the patient whose patient information has been permitted to be disclosed from the recording device 304, and transmits the patient information to the worker terminal 200 of the worker to whom the patient information has been permitted to be disclosed.

When having received the patient information from the information management apparatus 300 in the worker terminal 200, the control device 204 displays the received patient information on the touchscreen 201. Consequently, the patient can disclose his/her own personal information to the worker, and the patient's personal information can be shared between the workers.

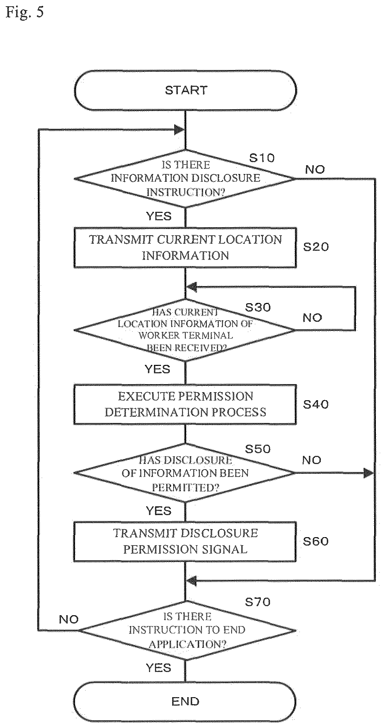

Fig. 5 is a flowchart illustrating the flow of processes to be executed by the patient terminal 100. The processes illustrated in Fig. 5 are executed by the control device 104 as a program that is started when the patient instructs the execution of the application aimed at the patient on the patient terminal 100 as described above. It is assumed in Fig. 5 that the above-mentioned patient information, hospital information, care provider information, health care worker and care worker information are recorded in advance in the recording device 304 of the information management apparatus 300. Moreover, it is assumed in Fig. 5 that as described above, the login process performed by transmitting the login requirement information to the information management apparatus 300 is already completed, and the patient's use of the application aimed at the patient has been permitted.

In Step S10, the control device 104 judges whether or not to have detected that the patient has performed the operation for an information disclosure instruction as described above. If it is judged to be negative in Step S10, processing proceeds to Step S70 described below. Conversely, if it is judged to be positive in Step S10, processing proceeds to Step S20.

In Step S20, the control device 104 identifies the current location, for example, the latitude and longitude, of the patient terminal 100 based on GPS signals received by the GPS module 106, and transmits information indicating the identified current location to the information management apparatus 300. Processing then proceeds to Step S30.

In Step S30, the control device 104 judges whether or not to have received information indicating the current location of the worker terminal 200 being the closest to the patient terminal 100 from the information management apparatus 300. If it is judged to be positive in Step S30, processing proceeds to Step S40.

In Step S40, the control device 104 executes the above-mentioned permission determination process steps (A) to (G) based on the current location of the patient terminal 100 and the current location of the worker terminal 200, and executes the permission determination process for determining whether or not to permit the worker who carries the relevant worker terminal 200 as the disclosure destination of the personal information, that is, the patient information, of the patient. Processing then proceeds to Step S50.

In Step S50, the control device 104 judges whether or not to have determined to permit the disclosure of the patient information to the worker targeted for the permission determination process, based on the execution results of the permission determination process steps (A) to (G). If it is judged to be negative in Step S50, processing proceeds to Step S70 described below. Conversely, if it is judged to be positive in Step S50, processing proceeds to Step S60.

In Step S60, the control device 104 transmits, to the information management apparatus 300, the disclosure permission information indicating that the patient information has been permitted to be disclosed to the worker targeted for the permission determination process. Processing then proceeds to Step S70.

In Step S70, the control device 104 judges whether or not the patient has instructed the end of the application aimed at the patient. It is assumed that the patient can instruct the end of the application aimed at the patient by performing a predetermined operation, for example, touching an end button displayed on the touchscreen 101. If it is judged to be negative in Step S70, processing returns to Step S10. Conversely, if it is judged to be positive in Step S70, processing ends.

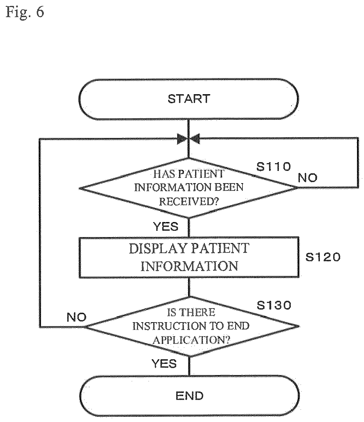

Fig. 6 is a flowchart illustrating the flow of processes to be executed by the worker terminal 200. The processes illustrated in Fig. 6 are executed by the control device 204 as a program that is started when the worker instructs the execution of the application aimed at the worker on the worker terminal 200 as described above. It is assumed in Fig. 6 that the above-mentioned patient information, hospital information, care provider information, and health care worker and care worker information are recorded in advance in the recording device 304 of the information management apparatus 300. Moreover, it is assumed in Fig. 6 that as described above, the login process performed by transmitting the login requirement information to the information management apparatus 300 is already completed, the worker's use of the application aimed at the worker has been permitted, and the control device 204 transmits information indicating the current location of the worker terminal 200 to the information management apparatus 300 at preset predetermined time intervals.

In Step S110, the control device 204 judges whether or not to have received the patient information from the information management apparatus 300. If it is judged to be positive in Step S110, processing proceeds to Step S120.

In Step S120, the control device 204 displays, on the touchscreen 201, the patient information received from the information management apparatus 300. Processing then proceeds to Step S130.

In Step S130, the control device 204 judges whether or not the worker has instructed the end of the application aimed at the worker. It is assumed that the worker can instruct the end of the application aimed at the worker by performing a predetermined operation, for example, touching an end button displayed on the touchscreen 201. If it is judged to be negative in Step S130, processing returns to Step S110. Conversely, if it is judged to be positive in Step S130, processing ends.

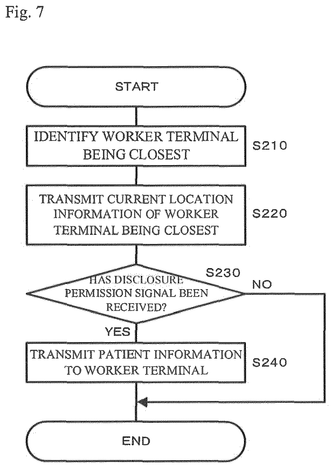

Fig. 7 is a flowchart illustrating the flow of processes to be executed by the information management apparatus 300 according to the embodiment. The processes illustrated in Fig. 7 are executed by the control device 303 as a program that is started upon receipt of the information indicating the current location transmitted in Step S20 of Fig. 5 from the patient terminal 100. It is assumed in Fig. 7 that the above-mentioned patient information, hospital information, care provider information, and health care worker and care worker information are recorded in advance in the recording device 304 of the information management apparatus 300. Moreover, whenever receiving information indicating the current location of each worker terminal 200 transmitted from the worker terminal 200 at the predetermined time intervals, the control device 303 records the received latest information indicating the current location in the buffer memory or recording device 304. Moreover, it is assumed in Fig. 7 that as described above, the authentication process is already completed based on the login requirement information received from the patient terminal 100 and the worker terminal 200, and the patient's use of the application aimed at the patient and the worker's use of the application aimed at the worker have been permitted.

In Step S210, the control device 303 identifies the worker terminal 200 being the closest to the patient terminal 100 based on the current location information received from the patient terminal 100 as described above. Processing then proceeds to Step S220.

In Step S220, the control device 303 transmits, to the patient terminal 100, information indicating the current location of the identified worker terminal 200 being the closest to the patient terminal 100. Processing then proceeds to Step S230.

In Step S230, the control device 303 judges whether or not to have received the above-mentioned disclosure permission information from the patient terminal 100. If it is judged to be negative in Step S230, processing ends. Conversely, if it is judged to be positive in Step S230, processing proceeds to Step S240.

In Step S240, the control device 303 reads, from the recording device 304, the patient information of the patient whose patient information has been permitted to be disclosed, and transmits the patient information to the worker terminal 200 of the worker to whom the patient information has been permitted to be disclosed. Processing then ends.

According to the embodiment described above, the following operations and effects can be obtained.
(1) The patient terminal 100 carried by the patient whose personal information is permitted to be disclosed to the worker is configured such that when having accepted a request of the patient to disclose the personal information to the worker, the control device 104 determines whether or not to permit the worker as a disclosure destination of the personal information based on the current location of the patient terminal 100 and the current location of the worker terminal 200, and if having determined to permit the worker as the disclosure destination of the personal information, transmits, to the information management apparatus 300, disclosure permission information indicating that the personal information of the patient has been permitted to be disclosed to the worker. The information management apparatus 300 is configured such that when having received the disclosure permission information from the patient terminal 100, the control device 303 reads the personal information of the patient from the recording device 304 and transmits the personal information to the worker terminal 200 of the worker to whom the personal information of the patient has been permitted to be disclosed. The worker terminal 200 carried by the worker involved in health care or care work is configured such that the control device 204 receives the personal information of the patient from the information management apparatus 300, and displays the received personal information of the patient on the touchscreen 201. Consequently, the patient can disclose the personal information to the workers of his/her own will. Accordingly, the patient's personal information can be shared between a plurality of workers. Moreover, whether or not to permit the worker as the disclosure destination of the personal information is determined based on the current location of the patient terminal and the current location of the worker terminal. Accordingly, security in the disclosure of the personal information can be increased.
(2) The patient terminal 100 is configured such that the control device 104 determines to permit the worker as the disclosure destination of the personal information if the current location of the patient terminal 100 and the current location of the worker terminal 200 are within the predetermined distance in the permission determination process step (A). Consequently, a worker being the closest to the patient can be permitted as the disclosure destination of the personal information. The security in the disclosure of the personal information can be increased.
(3) The patient terminal 100 is configured such that the control device 104 determines to permit the worker as the disclosure destination of the personal information if the current location of the patient terminal and the current location of the worker terminal are within a site of a preset medical institution in the permission determination process step (B). Consequently, only if the patient and the worker are within the site of the same medical institution, the worker can be permitted as the disclosure destination of the personal information. The security in the disclosure of the personal information can be increased.
(4) The patient terminal 100 is configured such that the control device 104 determines to permit the worker as the disclosure destination of the personal information if the carrier of the worker terminal 200 belongs the medical institution in the permission determination process step (C). Consequently, only if the worker is a person who belongs to the medical institution, the worker can be permitted as the disclosure destination of the personal information. The security in the disclosure of the personal information can be increased.
(5) The patient terminal 100 is configured such that the control device 104 determines to permit the worker as the disclosure destination of the personal information if the personal information is permitted to be disclosed to the carrier of the worker terminal 200 in the medical institution in the permission determination process step (D). Consequently, only if the worker is a person who belongs to the medical institution and is also permitted to be disclosed the personal information, the worker can be permitted as the disclosure destination of the personal information. The security in the disclosure of the personal information can be increased.
(6) The patient terminal 100 is configured such that the control device 104 determines to permit the worker as the disclosure destination of the personal information if the medical institution has a department corresponding to the specialty specified by the referral information in the permission determination process step (E). Consequently, only if the medical institution to which the worker belongs has a department that is necessary to treat the patient, the worker can be permitted as the disclosure destination of the personal information. The security in the disclosure of the personal information can be increased.
(7) The patient terminal 100 is configured such that the control device 104 determines to permit the worker as the disclosure destination of the personal information if the specialty specified by the referral information agrees with a specialty of the carrier of the worker terminal 200 in the permission determination process step (F). Consequently, only if the worker has expert knowledge specified by a worker of a referral source, the worker can be permitted as the disclosure destination of the personal information. The security in the disclosure of the personal information can be increased.
(8) The patient terminal 100 is configured such that the control device 104 determines to permit the worker as the disclosure destination of the personal information if the medical institution includes a facility in accordance with the purpose of the referral specified by the referral information in the permission determination process step (G). Specifically, it is configured such that when the purpose of the referral specified by the referral information is transfer, if the medical institution has an admission facility, the worker is determined to be permitted as the disclosure destination of the personal information. Moreover, it is configured such that when the purpose of the referral specified by the referral information is surgery, if the medical institution has a surgical facility, the worker is determined to be permitted as the disclosure destination of the personal information. Moreover, it is configured such that when the purpose of the referral specified by the referral information is photography, if the medical institution has a photographic facility, the worker is determined to be permitted as the disclosure destination of the personal information. Consequently, only if the medical institution to which the worker belongs has a facility expected by the worker of the referral source, the worker can be permitted as the disclosure destination of the personal information. The security in the disclosure of the personal information can be increased.

### - Modifications -

The information sharing system 10 according to the above-mentioned embodiment can also be modified as follows:
(1) In the above-mentioned embodiment, the permission determination process steps (B) to (G) of the permission determination process steps (A) to (G) assume a case where the worker is a health care worker. Accordingly, the example is described where the control device 104 of the patient terminal 100 judges whether or not to permit the disclosure of patient information based on the execution results of the permission determination process steps (A) to (G) if the worker is a health care worker, and judges whether or not to permit the disclosure of the patient information based only on the execution result of the permission determination process step (A) if the worker is a care worker. However, if the worker is a health care worker, at least one of the permission determination process steps (A) to (G) may be executed, or a different determination process step from the permission determination process steps (A) to (G) may be added. Moreover, if the worker is a care worker, a different determination process step from the permission determination process step (A) may be added.
(2) In the above-mentioned embodiment, the example is described where if having determined to permit the disclosure of information to a target worker based on the execution results of the permission determination process steps (A) to (G), the control device 104 of the patient terminal 100 transmits, to the information management apparatus 300, the disclosure permission information indicating that the patient information has been permitted to be disclosed to the worker targeted for the permission determination process. However, the control device 104 may be configured to, if having determined to permit the disclosure of the information to a target worker based on the execution results of the permission determination process steps (A) to (G), display, on the touchscreen 101, a confirmation screen that asks for consent to the disclosure of the personal information to the worker, and when the patient performs an operation indicating his/her consent on the confirmation screen, transmit the disclosure permission information to the information management apparatus 300. For example, it is configured such that a response to whether or not to consent to disclose the personal information to the worker can be given by touching a button of "YES" or "NO" on the confirmation screen. The control device 104 is simply required to transmit the disclosure permission information to the information management apparatus 300 when having detected that "YES" was touched on the confirmation screen. Consequently, the patient's consent can be obtained before the disclosure of the personal information to the worker.
(3) In the above-mentioned embodiment, the example is described where the health care worker and care worker information includes information on the permissions assigned to the worker, and information indicating the patient information view and edit permissions assigned to a health care worker by a medical institution to which the health care worker belongs, and information indicating the patient information view and edit permissions assigned to a care worker by a care institution to which the care worker belongs are recorded as the permissions assigned. However, it is not limited to the case where the view permission and the edit permission are set for the patient information. Whether or not to allow a view and whether or not to allow an edit may be set for each piece of information including the basic information, the life information, the treatment information, the handoff information, and the care and nursing information, which are included in the patient information.
   In this case, the view permission and edit permission for each piece of the information included in the patient information may be set according to the qualification held such as health care, nursing, or care, or an institution to which the worker belongs. For example, the view and edit permissions for the basic information, the life information, the treatment information, the handoff information, and the care and nursing information are simply required to be assigned to a doctor. It may be configured to assign a nurse the view permission for the basic information, the life information, the treatment information, the handoff information, and the care and nursing information, and also the edit permission for the basic information, the life information, and the care and nursing information. Moreover, it is simply required to assign a clerk of an institution to which the doctor belongs the view permission for the basic information, the life information, the treatment information, the handoff information, and the care and nursing information, and the edit permission for the basic information and the life information. It is simply required to assign a caregiver the view permission for the basic information, the life information, and the care and nursing information, and the edit permission for the basic information, the life information, and the care information. It is simply required to assign a clerk of an institution to which the caregiver belongs the view permission for the basic information, the life information, and the care and nursing information, and the edit permission for the basic information and the life information.
(4) In the above-mentioned embodiment, the patient terminal 100 is configured such that the control device 104 identifies the current location of the patient terminal 100 based on GPS signals received by the GPS module 106. Moreover, the worker terminal 200 is configured such that the control device 204 identifies the current location of the worker terminal 200 based on GPS signals received by the GPS module 206. However, the method for identifying the current location of the patient terminal 100 and the current location of the worker terminal 200 is not limited to this. For example, the current locations of the patient terminal 100 and the worker terminal 200 may be identified through another known method, for example, a method that uses information on an access point of a wireless LAN or a method that uses information on a base station of a mobile phone.
(5) In the above-mentioned embodiment, the example is described where general information terminals such as information terminals, for example, smartphones and tablet terminals, are used for the patient terminal 100 and the worker terminal 200. However, instead of these versatile information terminals, terminals designed specifically for communicating with the information management apparatus 300 may be used.
(6) In the above-mentioned embodiment, the patient terminal 100 and the worker terminal 200 are assumed to be smartphones including a communication function, and accordingly include a microphone for inputting the voice of a person who speaks. However, if devices that do not require the input of voice are used as the patient terminal 100 and the worker terminal 200, the microphone is not necessarily required to be mounted. Similarly, if devices that do not require the output of voice are used as the patient terminal 100 and the worker terminal 200, a speaker is not necessarily required to be mounted.
(7) In the above-mentioned embodiment, the example is described where the program of the application aimed at the patient is recorded in the nonvolatile memory forming the control device 104 included in the patient terminal 100, and the program of the application aimed at the worker is recorded in the nonvolatile memory forming the control device 204 included in the worker terminal 200. However, if the patient terminal 100 and the worker terminal 200 can use external memory such as a memory card, the program of the application aimed at the patient and the program of the application aimed at the worker may be recorded in the external memory.
(8) In the above-mentioned embodiment, the information management apparatus 300 includes the operating member 301 for being operated by an operator. However, if the operator remotely operates the information management apparatus 300 from a terminal connected via a communications line, the operating member 301 is not necessarily required to be included.

The present invention is not at all limited to the configuration of the above-mentioned embodiment unless the characteristic functions of the present invention are damaged. Moreover, it may be configured to combine the above-mentioned embodiment and a plurality of the modifications.

The content of the disclosure of the following priority basic application is incorporated herein as a citation.

Japanese Patent Application Number 2014-146828 (filed on July 17, 2014)

### DESCRIPTION OF REFERENCE SIGNS

- 10: Information sharing system
- 100: Patient terminal
- 101: Touchscreen
- 102: Communication module
- 103: Microphone
- 104: Control device
- 105: Speaker
- 106: GPS module
- 200: Worker terminal
- 201: Touchscreen
- 202: Communication module
- 203: Microphone
- 204: Control device
- 205: Speaker
- 206: GPS module
- 300: Information management apparatus
- 301: Operating member
- 302: Connection interface
- 303: Control device
- 304: Recording device

## Claims

1. An information sharing system (10) comprising:
a worker terminal (200) carried by a worker involved in health care or care work;
a mobile patient terminal (100) carried by a patient who permits the disclosure of his/her personal information to the worker; and
an information management apparatus (300) connected to the worker terminal (200) and the patient terminal (100) via a communications line, wherein
the mobile patient terminal (100) includes
a patient terminal current location identifying unit configured to identify the current location of the patient terminal (100),
a disclosure request accepting unit configured to accept a request of the patient to disclose the personal information to the worker,
a worker terminal current location identifying unit configured to, upon the disclosure request accepting unit accepting the disclosure request, receive information indicating the current location of the worker terminal (200) from the information management apparatus (300), and identify the current location of the worker terminal (200),
a permission determination unit (104) configured to determine whether or not to permit the worker as a disclosure destination of the personal information based on the current location of the patient terminal (100) identified by the patient terminal current location identifying unit and the current location of the worker terminal (200) identified by the worker terminal current location identifying unit, and
a disclosure permission information transmission unit configured to, upon the permission determination unit having determined to permit the worker as the disclosure destination of the personal information, transmit, to the information management apparatus (300), disclosure permission information indicating that the personal information of the patient has been permitted to be disclosed to the worker,
the information management apparatus (300) includes
a worker terminal current location information receiving unit configured to receive information indicating the current location of the worker terminal (200) from the worker terminal (200) at predetermined time intervals,
a worker terminal current location information transmission unit configured to transmit, to the patient terminal (100), the information indicating the current location of the worker terminal (200) received by the worker terminal current location information receiving unit, wherein a worker terminal current location information transmission unit transmits the information indicating the current location of the worker terminal (200) received by the worker terminal current location information receiving unit, when the current location of the worker terminal (200) is the closest location to the patient terminal (100),
a disclosure permission information receiving unit configured to receive the disclosure permission information from the patient terminal (100), and
a personal information transmission unit configured to, upon the disclosure permission information receiving unit having received the disclosure permission information, transmit the personal information of the patient recorded in a recording medium to the worker terminal (200) of the worker to whom the personal information of the patient has been permitted to be disclosed, and
the worker terminal (200) includes
a worker terminal current location identifying unit configured to identify the current location of the worker terminal (200) at predetermined time intervals, and transmit information indicating the identified current location to the information management apparatus (300),
a personal information receiving unit configured to receive the personal information of the patient from the information management apparatus (300), and
a personal information display unit configured to display the personal information of the patient received by the personal information receiving unit.

2. The information sharing system (10) according to claim 1, wherein when the current location of the patient terminal (100) and the current location of the worker terminal (200) are within a predetermined distance, the permission determination unit is configured to determine to permit the worker as the disclosure destination of the personal information.

3. The information sharing system (10) according to claim 1 or 2, wherein when the current location of the patient terminal (100) and the current location of the worker terminal (200) are located within a site of a preset medical institution, the permission determination unit is configured to determine to permit the worker as the disclosure destination of the personal information.

4. The information sharing system (10) according to claim 3, wherein when a carrier of the worker terminal (200) is a person who belongs to the medical institution, the permission determination unit is configured to determine to permit the worker as the disclosure destination of the personal information.

5. The information sharing system (10) according to claim 4, wherein when the personal information is permitted to be disclosed to the carrier of the worker terminal (200) in the medical institution, the permission determination unit is configured to determine to permit the worker as the disclosure destination of the personal information.

6. The information sharing system (10) according to claim 4 or 5, wherein
the personal information includes referral information for a doctor referring the patient to another doctor,
established specialty department identification-purpose information identifying specialty departments established in the medical institution to which the carrier of the worker terminal (200) belongs is recorded in the information management apparatus (300), and
the permission determination unit is configured to refer to the established specialty department identification-purpose information, and upon having determined that the medical institution has a department corresponding to a specialty specified by the referral information, is configured to determine to permit the worker as the disclosure destination of the personal information.

7. The information sharing system (10) according to claim 6, wherein
worker specialty department identification-purpose information identifying a specialty department of the carrier of the worker terminal (200) is recorded in the information management apparatus (300), and
the permission determination unit is configured to refer to the worker specialty department identification-purpose information, and upon having determined that a specialty department corresponding to the specialty specified by the referral information agrees with the specialty department of the carrier of the worker terminal (200), to determine to permit the worker as the disclosure destination of the personal information.

8. The information sharing system (10) according to claim 6 or 7, wherein when the medical institution includes a facility in accordance with the purpose of the referral specified by the referral information, the permission determination unit is configured to determine to permit the worker as the disclosure destination of the personal information.

9. The information sharing system (10) according to claim 8, wherein
admission facility possession/non-possession information indicating the possession/non-possession of an admission facility by the medical institution to which the carrier of the worker terminal (200) belongs is recorded in the information management apparatus (300), and
when the purpose of the referral specified by the referral information is transfer, the permission determination unit is configured to refer to the admission facility possession/non-possession information, and upon having determined that the medical institution has an admission facility, to determine to permit the worker as the disclosure destination of the personal information.

10. The information sharing system (10) according to claim 8 or 9, wherein
surgical facility possession/non-possession information indicating the possession/non-possession of a surgical facility by the medical institution to which the carrier of the worker terminal (200) belongs is recorded in the information management apparatus (300), and
when the purpose of the referral specified by the referral information is surgery, the permission determination unit is configured to refer to the surgical facility possession/non-possession information, and upon having determined that the medical institution has a surgical facility, to determine to permit the worker as the disclosure destination of the personal information.

11. The information sharing system (10) according to any one of claims 8 to 10, wherein
photographic facility possession/non-possession information indicating the possession/non-possession of a photographic facility by the medical institution to which the carrier of the worker terminal (200) belongs is recorded in the information management apparatus (300), and
when the purpose of the referral specified by the referral information is photography, the permission determination unit is configured to refer to the photographic facility possession/non-possession information, and upon having determined that the medical institution has a photographic facility, to determine to permit the worker as the disclosure destination of the personal information.

12. The information sharing system (10) according to any one of claims 1 to 11, wherein
the patient terminal (100) further includes a consent confirmation unit for, when the permission determination unit has determined to permit the worker as the disclosure destination of the personal information, confirming the patient's consent to the disclosure of the personal information to the worker, and
when the consent confirmation unit has confirmed the patient's consent to the disclosure of the personal information, the disclosure permission information transmission unit is configured to transmit the disclosure permission information to the information management apparatus (300).

13. The mobile patient terminal (100) according to any one of claims 1 to 12.

14. The information management apparatus (300) according to any one of claims 1 to 12.

## Patentansprüche

1. Ein Informationsaustauschsystem (10), das folgende Merkmale aufweist:
ein Mitarbeiterterminal (200), das von einem Mitarbeiter getragen wird, der im Gesundheitswesen oder Pflegewesen tätig ist;
ein mobiles Patiententerminal (100), das von einem Patienten getragen wird, der die Offenlegung seiner persönlichen Informationen an den Mitarbeiter genehmigt; und
eine Informationsverwaltungsvorrichtung (300), die mit dem Mitarbeiterterminal (200) und dem Patiententerminal (100) über eine Kommunikationsleitung verbunden ist, wobei
das mobile Patiententerminal (100) Folgendes umfasst:
eine Einheit zur Identifizierung des aktuellen Orts des Patiententerminals, die dazu konfiguriert ist, den aktuellen Ort des Patiententerminals (100) zu identifizieren,
eine Einheit zum Akzeptieren einer Offenlegungsanforderung, die dazu konfiguriert ist, eine Anforderung des Patienten, die persönlichen Informationen dem Mitarbeiter offenzulegen, zu akzeptieren,
eine Einheit zur Identifizierung des aktuellen Orts des Mitarbeiterterminals, die dazu konfiguriert ist, nach einem Akzeptieren der Offenlegungsanforderung seitens der Einheit zum Akzeptieren einer Offenlegungsanforderung, Informationen, die den aktuellen Ort des Mitarbeiterterminals (200) angeben, von der Informationsverwaltungsvorrichtung (300) zu empfangen und den aktuellen Ort des Mitarbeiterterminals (200) zu identifizieren,
eine Genehmigungsbestimmungseinheit (104), die dazu konfiguriert ist, zu bestimmen, ob der Mitarbeiter als Ziel der Offenlegung der persönlichen Informationen genehmigt werden soll oder nicht, basierend auf dem aktuellen Ort des Patiententerminals (100), der durch die Einheit zur Identifizierung des aktuellen Orts des Patiententerminals identifiziert wird, und dem aktuellen Ort des Mitarbeiterterminals (200), der durch die Einheit zur Identifizierung des aktuellen Orts des Mitarbeiterterminals identifiziert wird, und
eine Einheit zum Senden von Offenlegungsgenehmigungsinformationen, die dazu konfiguriert ist, nach einem Bestimmen durch die Genehmigungsbestimmungseinheit (104), dass der Mitarbeiter als Ziel der Offenlegung der persönlichen Informationen zu genehmigen ist, Offenlegungsgenehmigungsinformationen, die angeben, dass genehmigt wurde, die persönlichen Informationen des Patienten dem Mitarbeiter offenzulegen, an die Informationsverwaltungsvorrichtung (300) zu senden
wobei die Informationsverwaltungsvorrichtung (300) Folgendes umfasst:
eine Einheit zum Empfangen von Informationen über den aktuellen Ort des Mitarbeiterterminals, die dazu konfiguriert ist, Informationen, die den aktuellen Ort des Mitarbeiterterminals (200) angeben, in vorbestimmten Zeitintervallen von dem Mitarbeiterterminal (200) zu empfangen,
eine Einheit zum Senden von Informationen über den aktuellen Ort des Mitarbeiterterminals, die dazu konfiguriert ist, die Informationen, die den aktuellen Ort des Mitarbeiterterminals (200) angeben, die durch die Einheit zum Empfangen von Informationen über den aktuellen Ort des Mitarbeiterterminals empfangen werden, an das Patiententerminal (100) zu senden, wobei eine Einheit zum Senden von Informationen über den aktuellen Ort des Mitarbeiterterminals die Informationen sendet, die den aktuellen Ort des Mitarbeiterterminals (200) angeben, die durch die Einheit zum Empfangen von Informationen über den aktuellen Ort des Mitarbeiterterminals empfangen werden, wenn der aktuelle Ort des Mitarbeiterterminals (200) der nächstgelegene Ort zu dem Patiententerminal (100) ist,
eine Einheit zum Empfangen von Offenlegungsgenehmigungsinformationen, die dazu konfiguriert ist, die Offenlegungsgenehmigungsinformationen von dem Patiententerminal (100) zu empfangen, und
eine Einheit zum Senden von persönlichen Informationen, die dazu konfiguriert ist, nach einem Empfangen der Offenlegungsgenehmigungsinformationen seitens der Einheit zum Empfangen von Offenlegungsgenehmigungsinformationen, die persönlichen Informationen des Patienten, die in einem Aufzeichnungsmedium aufgezeichnet sind, an das Mitarbeiterterminal (200) des Mitarbeiters zu senden, für den genehmigt wurde, dass ihm die persönlichen Informationen des Patienten offengelegt werden, und
wobei das Mitarbeiterterminal (200) Folgendes umfasst:
eine Einheit zur Identifizierung des aktuellen Orts des Mitarbeiterterminals, die dazu konfiguriert ist, den aktuellen Ort des Mitarbeiterterminals (200) in vorbestimmten Zeitintervallen zu identifizieren und Informationen, die den identifizierten aktuellen Ort angeben, an die Informationsverwaltungsvorrichtung (300) zu senden,
eine Einheit zum Empfangen von persönlichen Informationen, die dazu konfiguriert ist, die persönlichen Informationen des Patienten von der Informationsverwaltungsvorrichtung (300) zu empfangen, und
eine Einheit zum Anzeigen von persönlichen Informationen, die dazu konfiguriert, die persönlichen Informationen des Patienten anzuzeigen, die seitens der Einheit zum Empfangen von persönlichen Informationen empfangen werden.

2. Das Informationsaustauschsystem (10) gemäß Anspruch 1, wobei, wenn der aktuelle Ort des Patiententerminals (100) und der aktuelle Ort des Mitarbeiterterminals (200) sich in einem vorbestimmten Abstand befinden, die Genehmigungsbestimmungseinheit zum Bestimmen konfiguriert ist, dass der Mitarbeiter als Ziel der Offenlegung der persönlichen Informationen genehmigt werden soll.

3. Das Informationsaustauschsystem (10) gemäß Anspruch 1 oder 2, wobei, wenn der aktuelle Ort des Patiententerminals (100) und der aktuelle Ort des Mitarbeiterterminals (200) sich innerhalb eines Standorts einer vorab festgelegten medizinischen Einrichtung befinden, die Genehmigungsbestimmungseinheit zum Bestimmen konfiguriert ist, dass der Mitarbeiter als Ziel der Offenlegung der persönlichen Informationen genehmigt werden soll.

4. Das Informationsaustauschsystem (10) gemäß Anspruch 3, wobei, wenn ein Träger des Mitarbeiterterminals (200) eine Person ist, die zu der medizinischen Einrichtung gehört, die Genehmigungsbestimmungseinheit zum Bestimmen konfiguriert ist, dass der Mitarbeiter als Ziel der Offenlegung der persönlichen Informationen genehmigt werden soll.

5. Das Informationsaustauschsystem (10) gemäß Anspruch 4, wobei, wenn genehmigt wurde, dass die persönlichen Informationen dem Träger des Mitarbeiterterminals (200) in der medizinischen Einrichtung offengelegt werden, die Genehmigungsbestimmungseinheit zum Bestimmen konfiguriert ist, dass der Mitarbeiter als Ziel der Offenlegung der persönlichen Informationen genehmigt werden soll.

6. Das Informationsaustauschsystem (10) gemäß Anspruch 4 oder 5, wobei
die persönlichen Informationen Überweisungsinformationen für einen Arzt umfassen, der den Patienten an einen anderen Arzt überweist,
Informationen zum Zweck der Identifizierung von eingerichteten Fachabteilungen, die Fachabteilungen identifizieren, die in der medizinischen Einrichtung eingerichtet sind, zu der der Träger des Mitarbeiterterminals (200) gehört, in der Informationsverwaltungsvorrichtung (300) aufgezeichnet sind, und
die Genehmigungsbestimmungseinheit zum Verweisen auf die Informationen zum Zweck der Identifizierung von eingerichteten Fachabteilungen und, nach einem Bestimmen, dass die medizinische Einrichtung eine Abteilung aufweist, die einer durch die Überweisungsinformationen festgelegten Fachabteilung entspricht, zum Bestimmen konfiguriert ist, dass der Mitarbeiter als Ziel der Offenlegung der persönlichen Informationen genehmigt werden soll.

7. Das Informationsaustauschsystem (10) gemäß Anspruch 6, wobei
Informationen zum Zweck der Identifizierung von Fachabteilungen des Mitarbeiters, die eine Fachabteilung des Trägers des Mitarbeiterterminals (200) identifizieren, in der Informationsverwaltungsvorrichtung (300) aufgezeichnet sind, und
die Genehmigungsbestimmungseinheit zum Verweisen auf die Informationen zum Zweck der Identifizierung von Fachabteilungen des Mitarbeiters und, nach einem Bestimmen, dass eine Fachabteilung der Fachabteilung entspricht, die durch die Überweisungsinformationen angegeben ist, mit der Fachabteilung des Trägers des Mitarbeiterterminals (200) übereinstimmt, zum Bestimmen konfiguriert ist, dass der Mitarbeiter als Ziel der Offenlegung der persönlichen Informationen genehmigt werden soll.

8. Das Informationsaustauschsystem (10) gemäß Anspruch 6 oder 7, wobei, wenn die medizinische Einrichtung eine Einrichtung gemäß dem Zweck der Überweisung umfasst, die durch die Überweisungsinformationen angegeben ist, die Genehmigungsbestimmungseinheit zum Bestimmen konfiguriert ist, dass der Mitarbeiter als Ziel der Offenlegung der persönlichen Informationen genehmigt werden soll.

9. Das Informationsaustauschsystem (10) gemäß Anspruch 8, wobei
Informationen über den Besitz/Nicht-Besitz einer Aufnahmeeinrichtung, die den Besitz/Nicht-Besitz einer Aufnahmeeinrichtung seitens der medizinischen Einrichtung angeben, zu der der Träger des Mitarbeiterterminals (200) gehört, in der Informationsverwaltungsvorrichtung (300) aufgezeichnet sind, und
wenn der Zweck der Überweisung, der durch die Überweisungsinformationen angegeben ist, ein Transfer ist, die Genehmigungsbestimmungseinheit zum Verweisen auf die Informationen über den Besitz/Nicht-Besitz einer Aufnahmeeinrichtung und, nach einem Bestimmen, dass die medizinische Einrichtung eine Aufnahmeeinrichtung aufweist, zum Bestimmen konfiguriert ist, dass der Mitarbeiter als Ziel der Offenlegung der persönlichen Informationen genehmigt werden soll.

10. Das Informationsaustauschsystem (10) gemäß Anspruch 8 oder 9, wobei Informationen über den Besitz/Nicht-Besitz einer chirurgischen Einrichtung, die den Besitz/Nicht-Besitz einer chirurgischen Einrichtung seitens der medizinischen Einrichtung angeben, zu der der Träger des Mitarbeiterterminals (200) gehört, in der Informationsverwaltungsvorrichtung (300) aufgezeichnet sind, und
wenn der Zweck der Überweisung, der durch die Überweisungsinformationen angegeben ist, ein chirurgischer Eingriff ist, die Genehmigungsbestimmungseinheit zum Verweisen auf die Informationen über den Besitz/Nicht-Besitz einer chirurgischen Einrichtung und, nach einem Bestimmen, dass die medizinische Einrichtung eine chirurgische Einrichtung aufweist, zum Bestimmen konfiguriert ist, dass der Mitarbeiter als Ziel der Offenlegung der persönlichen Informationen genehmigt werden soll.

11. Das Informationsaustauschsystem (10) gemäß einem der Ansprüche 8 bis 10, wobei
Informationen über den Besitz/Nicht-Besitz einer fotografischen Einrichtung, die den Besitz/Nicht-Besitz einer fotografischen Einrichtung seitens der medizinischen Einrichtung angeben, zu der der Träger des Mitarbeiterterminals (200) gehört, in der Informationsverwaltungsvorrichtung (300) aufgezeichnet sind, und
wenn der Zweck der Überweisung, der durch die Überweisungsinformationen angegeben ist, photographischer Art ist, die Genehmigungsbestimmungseinheit zum Verweisen auf die Informationen über den Besitz/Nicht-Besitz einer fotografischen Einrichtung und, nach einem Bestimmen, dass die medizinische Einrichtung eine photographische Einrichtung aufweist, zum Bestimmen konfiguriert ist, dass der Mitarbeiter als Ziel der Offenlegung der persönlichen Informationen genehmigt werden soll.

12. Das Informationsaustauschsystem (10) gemäß einem der Ansprüche 1 bis 11, wobei
das Patiententerminal (100) ferner eine Zustimmungsbestätigungseinheit aufweist, die, wenn die Genehmigungsbestimmungseinheit bestimmt hat, dass der Mitarbeiter als Ziel der Offenlegung der persönlichen Informationen genehmigt werden soll, die Zustimmung des Patienten bestätigt, dass die persönlichen Informationen dem Mitarbeiter offengelegt werden, und
wenn die Zustimmungsbestätigungseinheit die Zustimmung des Patienten zur Offenlegung der persönlichen Informationen bestätigt hat, die Einheit zum Senden von Offenlegungsgenehmigungsinformationen dazu konfiguriert ist, die Offenlegungsgenehmigungsinformationen an die Informationsverwaltungsvorrichtung (300) zu senden.

13. Das mobile Patiententerminal (100) gemäß einem der Ansprüche 1 bis 12.

14. Die Informationsverwaltungsvorrichtung (300) gemäß einem der Ansprüche 1 bis 12.

## Revendications

1. Système de partage d'informations (10), comprenant:
un terminal de travailleur (200) porté par un travailleur impliqué dans les soins de santé ou le travail de soins;
un terminal mobile de patient (100) porté par un patient qui permet la divulgation de ses informations personnelles au travailleur; et
un appareil de gestion d'informations (300) connecté au terminal de travailleur (200) et au terminal de patient (100) par l'intermédiaire d'une ligne de communication,
dans lequel
le terminal mobile de patient (100) comporte
une unité d'identification d'emplacement actuel de terminal de patient configurée pour identifier l'emplacement actuel du terminal de patient (100),
une unité d'acceptation de demande de divulgation configurée pour accepter une demande du patient de divulguer les informations personnelles au travailleur,
une unité d'identification d'emplacement actuel de terminal de travailleur configurée pour recevoir, lorsque l'unité d'acceptation de demande de divulgation accepte la demande de divulgation, les informations indiquant l'emplacement actuel du terminal de travailleur (200) de l'appareil de gestion d'informations (300), et pour identifier l'emplacement actuel du terminal de travailleur (200),
une unité de détermination d'autorisation (104) configurée pour déterminer s'il y a lieu ou non d'autoriser le travailleur comme destination de divulgation des informations personnelles sur base de l'emplacement actuel du terminal de patient (100) identifié par l'unité d'identification d'emplacement actuel de terminal de patient et de l'emplacement actuel de terminal de travailleur (200) identifié par l'unité d'identification d'emplacement actuel de terminal de travailleur, et
une unité de transmission d'informations d'autorisation de divulgation configurée pour transmettre, lorsque l'unité de détermination d'autorisation a décidé d'autoriser le travailleur comme destination de divulgation des informations personnelles, à l'appareil de gestion d'informations (300) les informations d'autorisation de divulgation indiquant qu'il a été autorisé de divulguer les informations personnelles du patient au travailleur, l'appareil de gestion d'informations (300) comporte
une unité de réception d'informations d'emplacement actuel de terminal de travailleur configurée pour recevoir les informations indiquant l'emplacement actuel du terminal de travailleur (200) du terminal de travailleur (200) à des intervalles de temps prédéterminés,
une unité de transmission d'informations d'emplacement actuel de terminal de travail configurée pour transmettre au terminal de patient (100) les informations indiquant l'emplacement actuel du terminal de travail (200) reçues par l'unité de réception d'informations d'emplacement actuel de terminal de travailleur, où une unité de transmission d'informations d'emplacement actuel de terminal de travailleur transmet les informations indiquant l'emplacement actuel du terminal de travailleur (200) reçues par l'unité de réception d'informations d'emplacement actuel de terminal de travailleur lorsque l'emplacement actuel du terminal de travailleur (200) est l'emplacement le plus proche du terminal de patient (100),
une unité de réception d'informations d'autorisation de divulgation configurée pour recevoir les informations d'autorisation de divulgation du terminal de patient (100), et
une unité de transmission d'informations personnelles configurée pour transmettre, lorsque l'unité de réception d'informations d'autorisation de divulgation a reçu les informations d'autorisation de divulgation, les informations personnelles du patient enregistrées sur un support d'enregistrement au terminal de travailleur (200) du travailleur auquel il a été autorisé de divulguer les informations personnelles du patient, et
le terminal de travailleur (200) comporte
une unité d'identification d'emplacement actuel de terminal de travailleur configurée pour identifier l'emplacement actuel du terminal de travailleur (200) à des intervalles de temps prédéterminés, et pour transmettre les informations indiquant l'emplacement actuel identifié à l'appareil de gestion d'informations (300),
une unité de réception d'informations personnelles configurée pour recevoir les informations personnelles du patient de l'appareil de gestion d'informations (300), et
une unité d'affichage d'informations personnelles configurée pour afficher les informations personnelles du patient reçues par l'unité de réception d'informations personnelles.

2. Système de partage d'informations (10) selon la revendication 1, dans lequel, lorsque l'emplacement actuel du terminal de patient (100) et l'emplacement actuel du terminal de travailleur (200) se trouvent dans les limites d'une distance prédéterminée, l'unité de détermination d'autorisation est configurée pour décider d'autoriser le travailleur comme destination de divulgation des informations personnelles.

3. Système de partage d'informations (10) selon la revendication 1 ou 2, dans lequel, lorsque l'emplacement actuel du terminal de patient (100) et l'emplacement actuel du terminal de travailleur (200) se trouvent dans un site d'un établissement médical préétabli, l'unité de détermination d'autorisation est configurée pour déterminer d'autoriser le travailleur comme destination de divulgation des informations personnelles.

4. Système de partage d'informations (10) selon la revendication 3, dans lequel, lorsqu'un porteur du terminal de travailleur (200) est une personne qui appartient à l'institution médicale, l'unité de détermination de permission est configurée pour déterminer d'autoriser le travailleur comme destination de divulgation des informations personnelles.

5. Système de partage d'informations (10) selon la revendication 4, dans lequel, lorsqu'il est autorisé que les informations personnelles soient divulguées au transporteur du terminal de travailleur (200) dans l'établissement médical, l'unité de détermination d'autorisation est configurée pour déterminer d'autoriser le travailleur comme destination de divulgation des informations personnelles.

6. Système de partage d'informations (10) selon la revendication 4 ou 5, dans lequel
les informations personnelles comportent des informations de référence pour un médecin qui renvoie le patient à un autre médecin,
les informations à des fins d'identification de services spécialisés établis identifiant les services spécialisés établis dans l'institution médicale à laquelle appartient le porteur du terminal de travailleur (200) sont enregistrées dans l'appareil de gestion d'informations (300), et
l'unité de détermination d'autorisation est configurée pour se référer aux informations à des fins d'identification de services spécialisés établis et, après avoir déterminé que l'établissement médical présente un service correspondant à une spécialité spécifiée par les informations de référence, est configurée pour déterminer d'autoriser le travailleur comme destination de divulgation des informations personnelles.

7. Système de partage d'informations (10) selon la revendication 6, dans lequel
les informations à des fins d'identification de services spécialisés de travailleurs identifiant un service spécialisé du porteur du terminal de travailleur (200) sont enregistrées dans l'appareil de gestion d'informations (300), et
l'unité de détermination d'autorisation est configurée pour se référer aux informations à des fins d'identification de services spécialisés de travailleurs et après avoir déterminé qu'un service spécialisé correspondant à la spécialité spécifiée par les informations de référence coïncide avec le service spécialisé du porteur du terminal de travailleur (200), pour déterminer d'autoriser le travailleur comme destination de divulgation des informations personnelles.

8. Système de partage d'informations (10) selon la revendication 6 ou 7, dans lequel, lorsque l'établissement médical comporte une installation conforme aux fins de la référence spécifiée par les informations de référence, l'unité de détermination d'autorisation est configurée pour déterminer d'autoriser le travailleur comme destination de divulgation des informations personnelles.

9. Système de partage d'informations (10) selon la revendication 8, dans lequel
les informations de possession/non-possession d'une installation d'admission indiquant la possession/non-possession d'une installation d'admission par l'établissement médical auquel appartient le porteur du terminal de travailleur (200) sont enregistrées dans l'appareil de gestion d'informations (300), et
lorsque les fins de la référence spécifiée par les informations de référence sont le transfert, l'unité de détermination d'autorisation est configurée pour se référer aux informations de possession/non-possession de l'installation d'admission et, après avoir déterminé que l'établissement médical présente une installation d'admission, pour déterminer d'autoriser le travailleur comme destination de divulgation des informations personnelles.

10. Système de partage d'informations (10) selon la revendication 8 ou 9, dans lequel
les informations de possession/non-possession d'une installation chirurgicale indiquant la possession/non-possession d'une installation chirurgicale par l'établissement médical auquel appartient le porteur du terminal de travailleur (200) sont enregistrées dans l'appareil de gestion d'informations (300), et
lorsque les fins de la référence spécifié par les informations de référence sont la chirurgie, l'unité de détermination d'autorisation est configurée pour se référer aux informations de possession/non-possession de l'installation chirurgicale et, après avoir déterminé que l'établissement médical présente une installation chirurgicale, pour déterminer d'autoriser le travailleur comme destination de divulgation des informations personnelles.

11. Système de partage d'informations (10) selon l'une quelconque des revendications 8 à 10, dans lequel
les informations de possession/non-possession d'une installation photographique indiquant la possession/non-possession d'une installation photographique par l'établissement médical auquel appartient le porteur du terminal de travailleur (200) sont enregistrées dans l'appareil de gestion d'informations (300), et
lorsque les fins de la référence spécifiée par les informations de référence sont la photographie, l'unité de détermination d'autorisation est configurée pour se référer aux informations de possession/non-possession de l'installation photographique et, après avoir déterminé que l'établissement médical présente une installation photographique, pour déterminer d'autoriser le travailleur comme destination de divulgation des informations personnelles.

12. Système de partage d'informations (10) selon l'une quelconque des revendications 1 à 11, dans lequel
le terminal de patient (100) comporte par ailleurs une unité de confirmation de consentement destinée à confirmer, lorsque l'unité de détermination d'autorisation a décidé d'autoriser le travailleur comme destination de divulgation des informations personnelles, le consentement du patient pour la divulgation des informations personnelles au travailleur, et
lorsque l'unité de confirmation de consentement a confirmé le consentement du patient pour la divulgation des informations personnelles, l'unité de transmission d'informations d'autorisation de divulgation est configurée pour transmettre les informations d'autorisation de divulgation à l'appareil de gestion d'informations (300).

13. Terminal mobile de patient (100) selon l'une quelconque des revendications 1 à 12.

14. Appareil de gestion d'informations (300) selon l'une quelconque des revendications 1 à 12.
